(19) **Europäisches Patentamt**
**European Patent Office**
**Office européen des brevets**

(11) **EP 4 670 753 A1**

(12) **EUROPEAN PATENT APPLICATION**
published in accordance with Art. 153(4) EPC

(43) Date of publication:
**31.12.2025 Bulletin 2026/01**

(21) Application number: **24760394.7**

(22) Date of filing: **21.02.2024**

(51) International Patent Classification (IPC):
**A61M 1/18** $^{(2006.01)}$   **A61M 1/36** $^{(2006.01)}$
**B01D 63/02** $^{(2006.01)}$   **B01D 67/00** $^{(2006.01)}$
**B01D 69/02** $^{(2006.01)}$   **B01D 69/08** $^{(2006.01)}$
**B01D 71/80** $^{(2006.01)}$

(52) Cooperative Patent Classification (CPC):
**A61M 1/36; B01D 63/02; B01D 67/00; B01D 69/02;**
**B01D 69/08; B01D 71/80**

(86) International application number:
**PCT/JP2024/006168**

(87) International publication number:
**WO 2024/177096 (29.08.2024 Gazette 2024/35)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **21.02.2023  JP 2023024859**

(71) Applicant: **Toray Industries, Inc.**
**Tokyo 103-8666 (JP)**

(72) Inventors:
• **YAMASHITA, Kyohei**
  **Otsu-shi, Shiga 520-8558 (JP)**
• **TAKAHASHI, Hiroshi**
  **Otsu-shi, Shiga 520-8558 (JP)**
• **SEKIYA, Yumiko**
  **Otsu-shi, Shiga 520-8558 (JP)**

(74) Representative: **Kador & Partner Part mbB**
**Corneliusstraße 15**
**80469 München (DE)**

(54) **HOLLOW FIBER MEMBRANE AND HOLLOW FIBER MEMBRANE MODULE**

(57)    Abstract: An object of the present invention is to provide a hollow fiber membrane that achieves both inhibiting adhesion of blood coagulation components to the surface of the membrane and having the high-efficiency performance of adsorptively removing inflammatory cells, inflammatory cytokines, and the like. The present invention provides a hollow fiber membrane including an anti-coagulant, and having a surface that comes into contact with a liquid to be treated, wherein the surface has an arithmetic-average roughness (Ra) of 0.10 to 0.80 $\mu$m in the radial direction.

Fig.1

## Description

Technical Field

The present invention relates to a hollow fiber membrane and a hollow fiber membrane module.

Background Art

A hollow fiber membrane is a cylindrical semipermeable membrane, and is generally known to have the function of allowing a substance to permeate selectively through penetrating micropores present in the surface of the membrane. Accordingly, a hollow fiber membrane is used for various applications in liquid-separating processes, for example, hemocatharsis applications, such as hemodialysis (HD) and hemofiltration (HF). In hemodialysis, a treatment is performed in such a manner that blood is made to pass through the inside of the hollow fiber membranes, and that water and a low-molecular-weight molecule, such as a urotoxia substance, in blood is taken out of the hollow fiber membranes through diffusion or filtration.

For example, Patent Literature 1 discloses a hollow fiber membrane composed of a copolymer of acrylonitrile and sodium methallylsulfonate, and containing polyethyleneimine, in which the hollow fiber membrane is surface-treated with an organic solvent to be thereby enabled to adsorb both inflammatory cytokines and inflammatory cells.

Patent Literature 2 discloses a hollow fiber membrane composed of a copolymer of acrylonitrile and sodium methallylsulfonate, which contains polyethyleneimine introduced into the surface thereof, and furthermore, on which surface heparin is then immobilized, whereby the hollow fiber membrane is enabled to inhibit blood from coagulating on the surface of the membrane, and simultaneously adsorb inflammatory cytokines.

Patent Literature 3 discloses a method of removing gram-negative bacteria, gram-positive bacteria, hepatitis virus, or the like from whole blood, using an adsorbent in which a polysaccharide containing heparin is immobilized via covalent binding on polymer beads or hollow fibers that have a cationized surface.

Prior Art References

Patent Literature

[0006]

　　Patent Literature 1: WO 2021/066152
　　Patent Literature 2: JP 2020-516424 A
　　Patent Literature 3: JP 2022-125040 A

Summary of Invention

Problem Which the Invention Tries to Solve

However, Patent Literature 1 describes nothing about an anti-coagulant. In cases where whole blood or blood having a low anti-coagulant concentration is made to pass the hollow fiber membranes, there is a concern that the blood may coagulate on the surface of the hollow fiber membrane.

A hollow fiber membrane according to Patent Literature 2 has an anti-coagulant immobilized in the surface thereof, thus repressing the coagulation of blood on the surface of the membrane. However, Patent Literature 2 describes nothing about the arithmetic-average roughness of the surface of the hollow fiber membrane, and the membrane is considered to be unable to adsorb inflammatory cells.

Polymer beads or hollow fibers disclosed in Patent Literature 3 have a cationized surface, and thus, has the possibility of adsorbing inflammatory cytokines. However, the Literature describes nothing about the arithmetic-average roughness of the surface, and the membrane is considered to be unable to adsorb inflammatory cells.

The antithrombogenic material disclosed in Patent Literature 4 is not intended to adsorb inflammatory cytokines and inflammatory cells.

In view of this, an object of the present invention is to provide a hollow fiber membrane that achieves both repressing adhesion of blood coagulation components to the surface of the membrane and having a high-efficiency performance of adsorptively removing inflammatory cells, inflammatory cytokines, and the like.

Means for Solving the Problem

The constitution of the present invention for solving the above-described problem is as follows.

(1) A hollow fiber membrane comprising an anti-coagulant, and having a surface that comes into contact with a liquid to be treated, wherein the surface has an arithmetic-average roughness (Ra) of 0.10 to 0.80 μm in the radial direction.
(2) The hollow fiber membrane according to (1), having an amount of the anti-coagulant per g of dry mass of 0.07 to 50.00 μmol.
(3) The hollow fiber membrane according to (1), having an amount of the anti-coagulant per g of dry mass of 0.27 to 30.00 μmol.
(4) The hollow fiber membrane according to any of (1) to (3), wherein the surface that comes in contact with the liquid to be treated comprises an amino group present therein.
(5) The hollow fiber membrane according to any one of (1) to (4), wherein the material of the hollow fiber membrane is a copolymer of: a hydrophobic monomer selected from the group consisting of acrylonitrile, methyl methacrylate, and styrene; and a hydrophilic monomer selected from the group consisting of methallylsulfonic acid, styrenesulfonic acid, and salts thereof.
(6) The hollow fiber membrane according to any one of (1) to (5), for use in hemocatharsis.
(7) The hollow fiber membrane according to any (1) to (6), for use in the adsorptive removal of activated leukocytes and inflammatory cytokines.
(8) A hollow fiber membrane module comprising the hollow fiber membrane according to any one of (1) to (7).
(9) The hollow fiber membrane module according to (8), for use in hemocatharsis.

Effects of Invention

A hollow fiber membrane according to the invention can inhibit adhesion of a blood coagulation component, and simultaneously remove inflammatory cells, inflammatory cytokines, and the like adsorptively with high efficiency.

Brief Description of Drawings

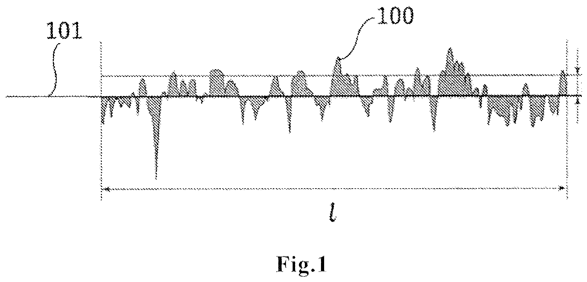

FIG. 1 is a diagram for explaining a method of determining an arithmetic-average roughness.

Description of Embodiments

A hollow fiber membrane according to the present invention is characterized by comprising an anti-coagulant, and having a surface that comes into contact with a liquid to be treated, wherein the surface has an arithmetic-average roughness (Ra) of 0.10 to 0.80 μm in the radial direction.
An "anti-coagulant" means a compound having a property of inhibiting blood coagulation that progresses, for example, owing to activation of a blood coagulation factor typified by thrombin.
Examples of the anti-coagulant include: aspirin, apixaban, argatroban, ethyl icosapentate, sarpogrelate hydrochloride, ticlopidine hydrochloride, edoxaban, ozagrel sodium, citric acid, trisodium citrate, clopidogrel sulfate, dipyridamole, cilostazol, dabigatran, dalteparin sodium, dextran sulfate, trapidil, parnaparin sodium, beraprost sodium, prasugrel sulfate, heparin (for example, heparin sodium, heparin potassium, and heparin calcium), low-molecular-weight heparin, heparin derivative such as acetylated heparin, polystyrenesulfonic acid, polyvinylsulfonic acid, nafamostat mesilate, rivaroxaban, limaprost alfadex, reviparin sodium, and warfarin potassium.
In cases where the anti-coagulant is packed in a hemocathartic column or added directly to blood, the anti-coagulant is preferably argatroban, citric acid, trisodium citrate, dextran sulfate, heparin, low-molecular-weight heparin, heparin derivative such as acetylated heparin, polystyrenesulfonic acid, polyvinylsulfonic acid, or nafamostat mesilate, from the viewpoint of safety of an organism. Among these, argatroban, trisodium citrate, heparin, low-molecular-weight heparin, and nafamostat mesilate, which have achieved lots of clinical results in extracorporeal circulation, are more preferable. The above-described anti-coagulants may be purified, or may be formed into a salt with sodium, potassium, or the like, and is not particularly limited, subject to being able to inhibit blood coagulation reaction.
Examples of a method of making a hollow fiber membrane to contain an anti-coagulant include: a method in which a solution containing an organic solvent having an anti-coagulant dissolved therein is made to pass through a module packed with hollow fiber membranes; and a method in which a hollow fiber membrane is immersed in a solution containing an organic solvent having an anti-coagulant dissolved therein.
A hollow fiber membrane according to the present invention preferably contains an amount of the anti-coagulant per g of dry mass of 0.07 to 50.00 μmol. From the viewpoint of sufficiently repressing coagulation of a blood component on the surface of the hollow fiber membrane, and reducing the risk of hemorrhage, the amount of the anti-coagulant contained per g of dry mass of the hollow fiber membrane is more preferably 0.17 to 45.00 $\mu$mol, still more preferably 0.21 to 40.00 $\mu$mol, particularly preferably 0.27 to 30.00 $\mu$mol, most preferably 0.30 to 30.00 $\mu$mol. Any of the preferable lower limits can be combined arbitrarily with any of the preferable upper limits.

The amount of the anti-coagulant contained in the hollow fiber membrane can be calculated by determining the concentration of the anti-coagulant in an extract liquid obtained by extracting the anti-coagulant from the hollow fiber membrane.

The amount of the anti-coagulant contained per g of dry mass of the hollow fiber membrane can be controlled in accordance with the kind of the organic solvent of, or the anti-coagulant concentration of, the solution containing the anti-coagulant, or in accordance with a linear velocity at which, a temperature at which, or a time during which, the hollow fiber membrane comes to contain the solution containing an anti-coagulant.

A method of measuring the anti-coagulant concentration of the extract liquid may be suitably selected in accordance with the kind of the anti-coagulant. For example, in cases where the anti-coagulant is heparin or a low-molecular-weight heparin, the concentration can be measured using Test Team Heparin S (manufactured by Sekisui Medical Co., Ltd.). In addition, for example, in cases where the anti-coagulant is nafamostat mesilate, argatroban, or trisodium citrate, the anti-coagulant concentration can be measured using a liquid chromatograph (HPLC). Specifically, the concentration can be measured using the method described in "Measurement of amount of anti-coagulant" below.

Examples of the solvent of a solution that contains an anti-coagulant, and is to be used to make the hollow fiber membrane contain the anti-coagulant, or examples of the solvent of an extract liquid for extracting an anti-coagulant include an organic solvent, water, salt-containing aqueous solution (for example, a phosphate buffer solution, borate buffer solution, ammonium chloride buffer solution, aqueous sodium hydroxide solution, physiological saline, aqueous sodium carbonate solution, or aqueous sodium hydrogen carbonate solution), and mixed solvents thereof. The hollow fiber membrane is insoluble or hardly soluble, and the anti-coagulant is soluble or easily soluble. Accordingly, a mixed solvent of an organic solvent with water or with an aqueous solution containing a salt is preferable.

Examples of the organic solvent include N,N-dimethylformamide, N,N-dimethylacetamide, dioxane, tetrahydrofuran, acetonitrile, acetone, and dimethylsulfoxide (hereinafter referred to as "DMSO"). Among these, DMSO, which has a high compatibility with the anti-coagulant and a high miscibility with water, is preferable. Any of the organic solvents may be used individually, or two or more kinds thereof may be combined and used in the form of a mixed solvent.

For example, in cases where, as the solvent mixture, a mixed solvent of DMSO with water or with a salt-containing aqueous solution is used, the concentration of DMSO is preferably 10 to 50 mass%. The same concentration applies also in cases where a mixed solvent of another solvent with water or with a salt-containing aqueous solution is used.

In cases where a solution containing an anti-coagulant is made to pass through the inside of the hollow fiber membranes to make the hollow fiber membrane contain the anti-coagulant, how easily the anti-coagulant is contained varies depending on the linear velocity. Accordingly, from the viewpoint of bringing, within a suitable range, the amount of the anti-coagulant to be contained in the hollow fiber membrane, the linear velocity is preferably 1.8 to 35.7 cm/minute, more preferably 5.3 to 26.7 cm/minute.

When the hollow fiber membrane is impregnated with the anti-coagulant, the temperature of a solution containing the anti-coagulant is preferably 10 to 80°C, more preferably 20 to 60°C, and the liquid passage time is preferably 5 minutes to 8 hours, preferably 15 minutes to 6 hours.

A "liquid to be treated" means a solution in which a component to be removed is dissolved, dispersed, or suspended. For example, in cases where the liquid to be treated is blood, examples of the component to be removed include cells or humoral factors in blood.

The "cells in blood" means cells contained in blood. Examples include: leukocyte components, such as granulocytes, monocytes, neutrophils, and eosinophils; erythrocytes; and platelets.

The "inflammatory cell" means a cell activated in blood by a protein, such as an inflammatory cytokine. Specific examples include activated leukocytes, activated platelets, and activated leukocyte/activated platelet complexes. In cases where a hollow fiber membrane according to the present invention is used in order to treat a patient with an inflammatory disease or a patient with a kidney disease in combination with an inflammatory disease, the substance to be removed is preferably activated leukocytes.

The "humoral factor in blood" means an organic substance dissolved in blood. Specific examples include: proteins, such as urea, $\beta$2-microglobulin, cytokine, IgE, and IgG; and polysaccharides, such as lipopolysaccharide (hereinafter referred to as an "LPS"). Among these, the substance to be removed is preferably a protein, such as a cytokine, or a polysaccharide, such as LPS, in the treatment of a patient with an inflammatory disease, and urea or $\beta$2-microglobulin in the treatment of a patient with a kidney disease. Furthermore, in the treatment of a patient with an inflammatory disease or a patient with a kidney disease in combination with an inflammatory disease, the substance to be removed is more preferably an inflammatory cytokine.

The term "inflammatory cytokine" means one group of proteins that are produced from various cells, such as immunocompetent cells, owing to a stimulus, such as an infection or an injury, and are released out of the cell, and act. Examples thereof include interferon-$\alpha$, interferon-$\beta$, interferon-$\gamma$, interleukin 1 to interleukin 15, tumor necrosis factor-$\alpha$,

tumor necrosis factor-$\beta$, high-mobility group box-1, erythropoietin, and monocyte chemotactic factor.

The term "adsorption" means a state where a substance adheres to a material, and is not easy to peel off, or a state of adsorption equilibrium. Examples of the principle of adsorption include: a state of adhesion by an intermolecular force, such as electrostatic interaction, hydrophobic interaction, hydrogen bonding, or a van der Waals force: and a state of physical adhesion by attachment of a cell, phagocytosis of a leukocyte, or the like.

The term "arithmetic-average roughness (hereinafter referred to as "Ra")" is an index for quantitating the smoothness of a surface, as standardized in JIS B 0601: 2001, and herein means an uneven state of a surface of a hollow fiber membrane in the radial direction, in which the surface comes into contact with a liquid to be treated. Specifically, Ra can be calculated from an image obtained by observing a surface of a hollow fiber membrane at a magnification of 50 times through an objective lens, using, for example, a laser microscope (for example, the ultradeep 3D-shape measuring microscope VK-9710, manufactured by Keyence Corporation) that is a laser confocal optical system, can perform two-dimensional scanning, and includes a line roughness analysis function (for example, the shape analysis application VK-H1A1/VK-H2A1, manufactured by Keyence Corporation).

In cases where the outer surface of the hollow fiber membrane is measured, the hollow fiber membrane preliminarily dried is directly placed on a sample table, and an image of the surface is imported. In cases where the inner surface is measured, the hollow fiber membrane preliminarily dried is cut out in semi-cylindrical form to expose the inner surface, and an image of the surface is imported. From the image obtained, 10 different positions are selected in random directions and places. At each position, the image along the reference length 1 is extracted in the radial direction of the hollow fiber membrane. From the image extracted along the reference length l, Ra can be calculated in accordance with the following Formula (1). FIG. 1 illustrates the reference length l extracted, a curved outline, and an average line. A value obtained by averaging the total of the absolute values of deviation from the average line of this extracted portion to the curved outline is Ra.

Here, f (x) means a function showing the uneven shape of the surface at an arbitrary position x in a laser-microscopic image. In this regard, Ra is herein calculated, assuming that the reference length l is 20 $\mu$m.

[Math. 1]

$$Ra = \frac{1}{\ell} \int_{0}^{\ell} \left| f(x) \right| \, dx \qquad \dots \text{Formula (1)}$$

When Ra is measured, the measurement values vary as the shape of the surface of the membrane varies depending on the hydration of the surface, or as the wet state of the hollow fiber membrane varies depending on the evaporation of water. Thus, the hollow fiber membrane needs to be dried in vacuo for 16 hours or more before measurement.

The term "average line" means a line described by fitting a curved outline to a straight line by the least-squares method, as standardized in JIS B 0601: 2001.

The term "curved outline" means a curved line described by contouring the surface of a material in an image of the surface as an object of measurement, in which the image is imported, using a laser microscope, as shown in FIG. 1. The curved outline is also referred as a measured cross-sectional curve.

Ra of the surface of the hollow fiber membrane that comes into contact with a liquid to be treated is 0.10 to 0.80 $\mu$m, preferably 0.20 to 0.70 $\mu$m, more preferably 0.30 to 0.60 $\mu$m, because making the surface sufficiently uneven makes it easier for a cell in blood to recognize a material, and because having a sufficient specific surface area enables the surface to adsorptively remove humoral factors in blood with high efficiency. Any of the preferable lower limits can be combined arbitrarily with any of the preferable upper limits.

The term "radial direction" of the hollow fiber membrane means the direction perpendicular to the longitudinal direction of the hollow fiber membrane, and is also referred to as the circumferential direction.

For example, roughness can be given to the surface of the hollow fiber membrane by bringing an organic solvent or an aqueous solution containing an organic solvent into contact with the surface of the hollow fiber membrane. In addition, Ra can be controlled, for example, in accordance with the kind, concentration, or temperature of the organic solvent to be used, or in accordance with a time during which, or a flow rate at which, the surface is brought into contact with the organic solvent.

Examples of the organic solvent to be used to give roughness to the surface of the hollow fiber membrane include: N,N-dimethylformamide; *N,N*-dimethylacetamide; dioxane; tetrahydrofuran; acetonitrile; acetone; DMSO; and mixed solvent of these with water; diethyl ether; toluene; xylene; nitrobenzene; chloroform; dichloromethane; carbon tetrachloride; hexane; and heptane.

Among these, N,N-dimethylformamide, N,N-dimethylacetamide, dioxane, tetrahydrofuran, acetonitrile, acetone, DMSO, or mixed solvent of these with water are preferable from the viewpoint of preventing the hollow fiber membrane from dissolving excessively. DMSO or a mixed solvent of DMSO with water is more preferable. A mixed solvent of DMSO with water is still more preferable. Two or more kinds of organic solvents may be combined and used in the form of a mixed solvent.

For example, in cases where, as the mixed solvent, a mixed solvent of DMSO with water is used, the concentration of DMSO is preferably 10 to 50 mass%. The same concentration applies also in cases where a mixed solvent of another solvent with water is used.

In cases where the organic solvent is brought into contact with the hollow fiber membrane to give roughness to the surface, the temperature of the organic solvent is preferably 10 to 80°C, more preferably 20 to 60°C.

Depending on the material of the hollow fiber membrane, there is concern that the hollow fiber membrane will dissolve excessively if in contact with the organic solvent for too long a time. On the other hand, if the hollow fiber membrane is in contact for too short a time, there is concern that sufficient roughness cannot be given to the surface of the hollow fiber membrane. Accordingly, the time during which the hollow fiber membrane is kept in contact with the organic solvent is preferably 30 minutes to 5 hours, more preferably 1 to 4 hours, still more preferably 1 to 3 hours.

In cases where the organic solvent is made to pass through the inside of the hollow fiber membranes to give roughness to the surface, there is similarly concern that the hollow fiber membrane will dissolve, depending on the material of the hollow fiber membrane. From the viewpoint of preventing the hollow fiber membrane from dissolving excessively, the linear velocity is preferably 1.8 to 35.7 cm/minute, more preferably 5.3 to 26.7 cm/minute. In addition, the liquid passage time is preferably 30 minutes to 5 hours, more preferably 1 to 4 hours, still more preferably 1 to 3 hours. The same time applies also in cases where the solvent is made to pass through the outside of the hollow fiber membranes.

In cases where the organic solvent is made to pass through the inside of the hollow fiber membranes to give roughness to the surface, the temperature of the organic solvent is preferably 10 to 80°C, more preferably 20 to 60°C. The same time applies also in cases where the solvent is made to pass through the outside of the hollow fiber membranes.

A hollow fiber membrane according to the present invention preferably has an amino group present in the surface which comes into contact with a liquid to be treated. The amino group on the surface of the hollow fiber membrane, which surface comes into contact with a liquid to be treated, may be derived from the material of the hollow fiber membrane, or may be derived from a ligand containing an amino group, which ligand is introduced into the surface of the hollow fiber membrane.

Examples of the "amino group" include: an amino group derived from a primary amine, such as methylamine, ethylamine, propylamine, butylamine, pentylamine, hexylamine, heptylamine, octylamine, or dodecylamine; an amino group derived from a secondary amine, such as methylhexylamine, diphenylmethylamine, or dimethylamine; an amino group derived from an amine having an unsaturated alkyl chain, such as allylamine; an amino group derived from a tertiary amine, such as trimethylamine, triethylamine, dimethylethylamine, phenyldimethylamine, or dimethylhexylamine; an amino group derived from an amine having an aromatic ring, such as 1-(3-aminopropyl)imidazole, pyridine-2-amine, or 3-sulfoaniline; and an amino group derived from a compound such as tris(2-aminoethyl)amine, ethylenediamine, diethylenetriamine, triethylenetetramine, tetraethylene pentamine, pentaethylenehexamine, hexaethyleneheptamine, heptaethyleneoctamine, octaethylenenonamine, dipropylenetriamine, polyethyleneimine, *N*-methyl-2,2'-diaminodiethylamine, *N*-acetylethylenediamine, or 1,2-bis(2-aminoethoxyethane), in which the compound is obtained by binding two or more amino groups (the compound is hereinafter referred to as a "polyamine") via an alkyl chain, aromatic compound, heterocyclic compound, carbocyclic compound, or the like.

Among these, an amino group derived from ethylenediamine, diethylenetriamine, triethylenetetramine, tetraethylene pentamine, pentaethylenehexamine, hexaethyleneheptamine, heptaethyleneoctamine, octaethylenenonamine, dipropylenetriamine, polyethyleneimine, N-methyl-2,2'-diaminodiethylamine, N-acetylethylenediamine, or 1,2-bis (2-aminoethoxyethane) is preferable, and an amino group derived from ethylenediamine, diethylenetriamine, triethylenetetramine, tetraethylene pentamine, polyethyleneimine, or polyethyleneimine is more preferable, from the viewpoints of being easier to dissolve in a solvent, having a structure having a reaction point for introduction into the hollow fiber membrane, and having high affinity for inflammatory cytokines. In this regard, the polyamine may be linear, branched, or cyclic.

In addition, the hydrogen atom bound to a basic nitrogen atom contained in the polyamine may be substituted, for example, with a substituent, which is, for example: a $C_{1-10}$ alkyl group; an unsaturated alkyl group, such as a vinyl group or an allyl group; an aryl group, such as a phenyl group, a naphthyl group, or an anthracyl group; or a heteroaryl group, such as an imidazolyl group, a pyridyl group, or a piperidyl group. Neither the number of substituents nor the position of substitution is particularly limited.

The term "ligand" means a compound bound to at least the surface of the hollow fiber membrane, in which the surface comes into contact with a liquid to be treated. In cases where the surface to come into contact with a liquid to be treated is the inner surface of the hollow fiber membrane, the ligand is bound to the inner surface. In cases where the surface to come into contact with a liquid to be treated is the outer surface of the hollow fiber membrane, the ligand is bound to the outer surface. The surface that does not come into contact with a liquid to be treated may be modified or unmodified with a ligand. In this regard, the ligand is preferably distributed in the whole of the surface that comes into contact with a liquid to be treated.

The ligand is not particularly limited to any chemical structure, subject to containing an amino group. Examples of a method of binding a ligand to the surface of the hollow fiber membrane include; a method in which the surface of the hollow fiber membrane and a ligand are bound by electrostatic interaction therebetween (for example, electrostatic interaction between an anionic functional group, such as a sulfonic group, and a cationic functional group, such as a polyamine); a method in which a predetermined functional group present in the surface of the hollow fiber membrane and a ligand are bound directly by covalent binding, hydrogen bonding, or the like; and a method in which a predetermined functional group present in the surface of the hollow fiber membrane and a ligand are bound via a spacer.

Examples of the structure of the spacer include a sulfonic group, amide group, carboxyl group, sulfonamide group, ureido group, and ether group. In cases where the hollow fiber membrane contains an anionic functional group (for example, a sulfonic group) as a constituent, a method in which the binding is due to electrostatic interaction is preferable, because the method makes it possible to simply introduce a ligand into the surface.

Examples of a method of binding a ligand to the surface of the hollow fiber membrane include: a method in which the hollow fiber membrane is immersed in a solution having an amino-group-containing compound dissolved therein; and a method in which a solution having an amino-group-containing compound dissolved therein is made to pass through the inside of the hollow fiber membranes.

Examples of a solvent in which an amino-group-containing compound is dissolved include water, a salt-containing aqueous solution (for example, a phosphate buffer solution, borate buffer solution, ammonium chloride buffer solution, aqueous sodium hydroxide solution, saline, aqueous sodium carbonate solution, or aqueous sodium hydrogen carbonate solution), and mixed solvents thereof. Water is preferable, because of not impeding the reaction of introduction into the hollow fiber membrane.

The pH of the solution having an amino-group-containing compound dissolved therein is preferably 7 to 12, and the concentration of the amino-group-containing compound in the solution is preferably 0.04 to 20 g/L.

The temperature of the solution, at which temperature the hollow fiber membrane is immersed in the solution having an amino-group-containing compound dissolved therein, is preferably 10 to 80°C, more preferably 20 to 60°C. The immersion time is preferably 30 minutes to 24 hours, preferably 1 to 8 hours.

The amount of the amino group in the surface of the hollow fiber membrane according to the present invention, which surface comes into contact with a liquid to be treated, is not particularly limited. From the viewpoint of giving sufficient performance for adsorbing an organic substance having an electric charge, such as a blood component, and from the viewpoint of suppressing variation of the pH of blood, the amount is preferably 0.05 to 1.00 mmol or less per g of dry mass of the hollow fiber membrane, more preferably 0.10 to 0.50 mmol, still more preferably 0.10 to 0.30 mmol. Any of the preferable lower limits can be combined arbitrarily with any of the preferable upper limits.

As the material of a hollow fiber membrane according to the present invention, for example, a known material to be used for hemocatharsis can be adopted. The hollow fiber membrane, if composed of only a water-soluble component, may be swollen by water in blood so that the micropores may be clogged. In addition, because of the easiness of introducing a ligand, the material of the hollow fiber membrane is preferably a copolymer of a hydrophobic monomer and a hydrophilic monomer.

The term "hydrophobic monomer" means a monomer that is polymerized to afford a polymer that exhibits insolubility to water.

To "exhibit insolubility to water" means that a dry-mass variation is 1% or less between before and after the polymer is placed in water. The dry-mass variation is given, as follows: a polymer is immersed at 37°C for 1 hour in water in an amount 9 times as large as the amount of the dry mass of the polymer; then, the polymer is pulled up using tweezers or the like; the remaining water is dried in vacuo at 50°C or less to leave a solid; and the ratio of the dry mass of the solid left to the dry mass of the polymer before the immersion is the dry-mass variation.

The "hydrophilic monomer" means a monomer that affords a polymer that exhibits solubility to water.

To "exhibit solubility to water" means that a dry-mass variation is 50% or more, in which the dry-mass variation is given, as follows: a polymer is immersed at 37°C for 1 hour in water in an amount 9 times as large as the amount of the dry mass of the polymer; then, the polymer is pulled up using tweezers or the like; the remaining water is dried in vacuo at 50°C or less to leave a solid; and the variation between the dry mass of the solid left and the dry mass of the polymer before the immersion is the dry-mass variation.

Examples of the material of the hollow fiber membrane include: a polymer or copolymer of a hydrophobic monomer(s) selected from the group consisting of ethylene, propylene, acrylonitrile, acrylates such as methyl acrylate and ethyl acrylate, methacrylates such as methyl methacrylate and ethyl methacrylate, and styrene; a polymer or copolymer of a hydrophilic monomer(s) selected from the group consisting of vinyl pyrrolidone, ethylene glycol, propylene glycol, vinyl alcohol, methallylsulfonic acid, styrenesulfonic acid, and salts thereof; a copolymer of the above-described hydrophobic monomer(s) and the above-described hydrophilic monomer(s); an aromatic polyether sulfone, such as polysulfone or polyether sulfone; an aromatic polyether ketone, such as polyether ketone or polyether ether ketone; a sulfonated aromatic polyether sulfone, such as sulfonated polysulfone or sulfonated polyether sulfone; polyamide, such as polyamideimide, nylon 6, or nylon 6,6; polyurethane; polyester; and cellulose acetate.

From the viewpoints of copolymerizability with a hydrophilic polymer and easiness of introduction of a ligand containing an amino group, the material of the hollow fiber membrane is preferably a copolymer of: a hydrophobic monomer(s) selected from the group consisting of acrylonitrile, methyl methacrylate, and styrene; and a hydrophilic monomer(s) selected from the group consisting of methallylsulfonic acid, styrenesulfonic acid, and salts thereof. The material is more preferably a copolymer of acrylonitrile and methallylsulfonic acid or a salt thereof. In this regard, to the extent of not impairing the effects of the present invention, the material may be copolymerized with another monomer, and a functional group may be introduced into the side chain.

As an example of an embodiment of the present invention, a method of producing a hollow fiber membrane containing an acrylonitrile/sodium methallylsulfonate copolymer as a constituent will be described in detail. However, the present invention is not limited to the example.

Acrylonitrile is added to sodium methallylsulfonate, and the resulting mixture is polymerized in DMSO to produce a polymer having an [η] of 3.2. Furthermore, the polymer is diluted to afford a raw spinning solution having a total polymer concentration of 13.0 mass%. Next, using a core-in-sheath type spinneret having an inner diameter of 0.25 mm and a slit width of 0.1 mm for a hollow fiber membrane, this raw spinning solution is discharged at a rate of 1.4 mL/minute from the sheath portion. An aqueous solution of 80 mass% DMSO is discharged as a coagulating liquid from the core portion. Then, the fibers discharged through the spinneret having a temperature of 50°C is made to pass for 80 mm in air (at room temperature), then guided into a coagulation bath of water at 30°C to be coagulated, washed in water at 35°C, and then wound up, affording a hollow fiber membrane.

From the viewpoint of enhancing the fiber-forming properties and the fiber strength, the ratio of acrylonitrile is preferably 60 to 95 mol% in production of the raw spinning solution. On the other hand, from the viewpoint of binding, to the surface, a ligand for sufficient adsorptive removal of a substance to be removed, the ratio of sodium methallylsulfonate is preferably 5 to 40 mol%.

The thickness of the hollow fiber membrane may be suitably determined in accordance with the application. For example, in cases where the hollow fiber membrane is used for extracorporeal circulation, the thickness of the hollow fiber membrane is preferably 20 to 80 μm, more preferably 30 to 70 μm, still more preferably 40 to 60 μm.

The inner diameter of the hollow fiber membrane may be suitably determined in accordance with the thickness and outer diameter of the hollow fiber membrane. For example, in cases where the hollow fiber membrane is used for extracorporeal circulation, the inner diameter of the hollow fiber membrane is preferably 150 to 400 μm, more preferably 200 to 300 μm.

To remove an object substance by membrane separation, the pore size of the surface of the hollow fiber membrane needs to be a pore size larger than the size of the object substance to be removed. Accordingly, although the pore size of the surface of the hollow fiber membrane depends on the size of the object substance, the pore size is commonly preferably 0.1 to 50 nm, more preferably 1 to 30 nm.

That surface of the hollow fiber membrane which comes into contact with a liquid to be treated may be the inner surface or the outer surface of the hollow fiber membrane. The surface that comes into contact with a liquid to be treated is, among others, preferably the inner surface of the hollow fiber membrane, because the inner surface provides a more uniform flow path for the liquid to be treated, and has a smaller number of dead spaces.

In cases where a hollow fiber membrane according to the present invention is packed into a module, the hollow fiber membrane may be a hollow fiber membrane bundle constituted by a plurality of hollow fiber membranes. In particular, in cases where extracorporeal circulation is performed for the purpose of treating a patient with an inflammatory disease, a hollow fiber membrane according to the present invention is suitably used as a carrier for adsorptive removal of activated leukocytes and/or inflammatory cytokines. In cases where the hollow fiber membrane module is used for a hemocathartic therapy, blood guided out of the body may be made to pass through the module directly, or the module may be used in combination with a plasma separation membrane or the like.

The term "inflammatory disease" means all the diseases by which an inflammatory reaction is initiated in the body. Examples thereof include systemic lupus erythematosus, malignant rheumatoid arthritis, multiple sclerosis, ulcerative colitis, Crohn's disease, drug-induced hepatitis, alcoholic hepatitis, hepatitis A, hepatitis B, hepatitis C, hepatitis D, or hepatitis E, sepsis (for example, gram-negative-bacteria-derived sepsis, gram-positive-bacteria-derived sepsis, culture-negative sepsis, or fungal sepsis), influenza, acute respiratory distress syndrome (ARDS), acute lung injury (ALI), pancreatitis, idiopathic pulmonary fibrosis (IPF), inflammatory enteritis (for example, ulcerative colitis or Crohn's disease), blood preparation transfusion, organ transplantation, organ transplantation after reperfusion damage, cholecystitis, cholangitis, and newborn blood group incompatibility. Among the inflammatory diseases, examples of a disease which causes a causative agent to be released in blood, and for which a therapeutic effect by hemocatharsis can be particularly expected include drug-induced hepatitis, alcoholic hepatitis, hepatitis A, hepatitis B, hepatitis C, hepatitis D, or hepatitis E, sepsis (for example, gram-negative-bacteria-derived sepsis, gram-positive-bacteria-derived sepsis, culture-negative sepsis, or fungal sepsis), influenza, acute respiratory distress syndrome, acute lung injury, pancreatitis, and idiopathic pulmonary fibrosis.

In cases where the number of hollow fiber membranes to be packed in a container is 100 to 200 or more in the present invention, more activated leukocytes can be removed, and the area of the membranes is increased to increase the water permeability, but on the other hand, the amount of blood held in the hollow fiber membranes is increased. Having 10 to 100 as the number of the hollow fiber membranes to be packed allows evaluation under conditions closer to clinical conditions, from the viewpoint of the amount of blood held.

Examples of a method of evaluating the hemocathartic performance of the hollow fiber membrane in the present invention include a method of measuring the ratio of adsorption of IL-8. IL-8 is one kind of inflammatory cytokine contained in a blood component. It is known that, in a patient with an inflammatory disease, the value of IL-8 is markedly high in a blood component in the case of a disease developed owing to bronchiolitis or viral infection in particular. Thus, IL-8 is suitable as a blood component for evaluating the hemocathartic performance. It can be judged that the higher the ratio of adsorption of IL-8 is, the higher the hemocathartic performance of the hollow fiber membrane is.

In addition, examples of another method of evaluating the hemocathartic performance of the hollow fiber membrane in the present invention include a method of evaluating the ratio of removal of activated leukocytes. Examples of a method of calculating the ratio of removal of activated leukocytes include a method in which the hollow fiber membrane is packed into a container having an inlet and an outlet; a liquid containing activated leukocytes is made to pass through the container; and, from the variation of the concentration between the inlet and the outlet, the ratio of removal is calculated.

From the viewpoint that an activated leukocyte is a cell, and involves variation of measurements of the ratio of removal, a case where the number of hollow fiber membranes packed in a container is 100 to 200 or more, and where the ratio of removal of activated leukocytes is 6% or more can be judged as a case where the removal is significant. However, there is a concern that the pores of the hollow fiber membrane, if caused to adsorb activated leukocytes excessively, may be clogged, so that the water permeability is decreased. Thus, the ratio of removal of activated leukocytes is preferably 80% or less. In cases where the number of the hollow fiber membranes packed is 10 to 100, the ratio of removal of activated leukocytes is preferably 2.5% or more and preferably 30% or less.

The term "water permeability" indicates the amount of passage of water that permeates the penetrating pores present in the surface of the hollow fiber membrane. As the pore size is larger or as the number of pores is larger, the passage of water increases, and thus, the water permeability of the hollow fiber membrane increases. In cases where the number of the hollow fiber membranes packed in a container is 100 to 200 or more, the water permeability is preferably 0.5 to 5.0 $L/h/m^2/mmHg$, more preferably 1.0 to 3.0 $L/h/m^2/mmHg$, still more preferably 1.0 to 1.5 $L/h/m^2/mmHg$. In cases where the number of the hollow fiber membranes packed in a container is 10 to 100, the water permeability is preferably 0.016 to 0.100 $L/h/m^2/mmHg$, more preferably 0.022 to 0.050 $L/h/m^2/mmHg$, still more preferably 0.025 to 0.030 $L/h/m^2/mmHg$. Any of the preferable lower limits can be combined arbitrarily with any of the preferable upper limits.

The sizes or number of the penetrating pores present in the surface of the hollow fiber membrane, and contributive to water permeability can be controlled in accordance with the composition of a raw spinning solution and the spinning conditions for membrane formation.

The term "ratio of maintenance of water permeability" can be determined by calculating the ratio of variation of water permeability between before and after a liquid to be treated is brought into contact with the surface of the hollow fiber membrane. Even after the hollow fiber membrane is brought into contact with a liquid to be treated, but when the water permeability of the membrane is not varied, the ratio of maintenance of water permeability is a high value. On the other hand, in cases where contact with a liquid to be treated causes the membrane to decrease in water permeability, the ratio of maintenance of water permeability is a low value. The ratio of maintenance of water permeability is preferably 20% or more, more preferably 30% or more, still more preferably 50% or more, most preferably 60% or more.

The ratio of maintenance of water permeability is decreased by adhesion of a blood coagulation component to the surface of the hollow fiber membrane through which the blood has been made to pass. That is, having a suitable amount as the amount of an anti-coagulant contained in the hollow fiber membrane makes it possible to suppress a blood coagulation component from adhering to the surface of the hollow fiber membrane, and to maintain the water permeability.

A hollow fiber membrane module according to the present invention is characterized in that a hollow fiber membrane according to the present invention is packed in a container, that is, , the module includes the hollow fiber membrane according to the present invention.

A hollow fiber membrane module according to the present invention can be used for a hemocathartic application in which a blood component of interest is purified or removed from a liquid containing the blood component and the like. For example, the hollow fiber membrane can be used for separation of a specific blood component. The module is suitably used for an application in which, among these blood components, cells in blood or humoral factors in blood are adsorptively removed. The module is particular suitably used as a hollow fiber membrane module for adsorptively removing activated leukocytes and/or inflammatory cytokines among others.

Regarding the shape of the container of the hollow fiber membrane module, any shape is acceptable as long as the container has an inlet portion and an outlet portion for a liquid containing a blood component and the like; an inlet portion and an outlet portion for a dialysate; and a case portion; wherein the hollow fiber membrane can be packed in the case portion. In one embodiment, hollow fiber membranes are cut to a desired length, a desired number of the membranes are bundled, and then, the resulting bundle is inserted into a cylindrical case. Then, both ends are temporarily capped, and a potting material is put into both end portions of the hollow fiber membranes. After the potting material is solidified, both end portions are cut so that both ends of the hollow fiber membranes can be opened. A header is attached to both ends of the case, and the header and the nozzle portion of the case are plugged to produce a hollow fiber membrane module. From the viewpoint of allowing a sufficient amount of dialysate to flow uniformly, and from the viewpoint of maintaining the performance of adsorptive removal of activated leukocytes and/or inflammatory cytokines, the packing ratio of the hollow fiber membranes (the total cross-sectional area of the packed hollow fiber membranes in the longitudinal direction, with respect to the cross-sectional area of the case portion in the longitudinal direction) is preferably 20 to 80%, still more preferably 30 to 70%.

In the hollow fiber membrane module, the hollow fiber membrane may be packed singly, or another hollow fiber membrane or any of various filling materials may be packed in combination. Examples of the filling material include; sheet-shaped fibers, such as knitted fabrics, woven fabrics, and nonwoven fabrics; membranes; particles; and hydrogels.

Examples

A hollow fiber membrane according to the present invention will be specifically described below with reference to Examples. The present invention is not limited to these Examples.

<Measurement of arithmetic-average roughness>

A hollow fiber membrane was cut out to a length of 5 cm, further cut out in semi-cylindrical form to expose the inner surface, and dried in vacuo at 25°C for 16 hours. The inner surface of the hollow fiber membrane dried was photographed as an image at a magnification of 50 times through an objective lens, using a laser microscope (the ultradeep 3D-shape measuring microscope VK-9710; manufactured by Keyence Corporation). From the curved outline of one hollow fiber membrane in the image obtained, the image along the reference length 1 of 20 $\mu$m was extracted at each of 10 random positions. Using the analysis software installed in VK-9710, Ra of the inner surface in the radial direction was measured (in accordance with JIS B 0601: 2001) by analysis in the line roughness mode. This operation was performed on three different images, and the average of the values of a total of 30 positions obtained from the images was determined to calculate Ra of the inner surface of the hollow fiber membrane in the radial direction. Here, a value rounded to two decimal places was used.

<Measurement of amount of anti-coagulant>

Into a glass-made screw tube, 15 mg of a dried hollow fiber membrane was inserted, 0.5 mL of DMSO and 1.5 mL of physiological saline were added, and the resulting mixture was left to stand at 40°C for 16 hours to obtain an extract liquid of the hollow fiber membrane. The extract liquids obtained from the below-described hollow fiber membranes 1 to 3, hollow fiber membranes 13 to 17, and hollow fiber membranes 27 to 28 were each allowed to react in accordance with the operating procedures described in the attached document of Test Team Heparin S (Sekisui Medical Co., Ltd.). The absorbance at 405 nm was measured using a microplate reader (model: SpectraMax M5; manufactured by Molecular Device Japan K.K.) to determine the heparin concentration of the extract liquid. In addition, measurements were made of the extract liquids obtained from the below-described hollow fiber membranes 4 to 11 and hollow fiber membranes 18 to 26 under the below-described conditions, using a liquid chromatograph (HPLC), to determine the anti-coagulant concentration of the extract liquid.

Device: Prominence (manufactured by Shimadzu Corporation)
Column: Agilent Zorbax Eclipse XDB C18 (4.6 mm $\times$ 75 mm $\times$ 3.5 $\mu$m)
Column temperature: 40°C
Measurement time: 30 minutes
Amount of injection: 10 $\mu$L
Nafamostat mesilate-measuring wavelength: 241 nm
Argatroban-measuring wavelength: 260 nm
Trisodium citrate-measuring wavelength: 217 nm
Flow rate: 1.0 mL/minute
Liquid-feeding conditions: mobile phase A, acetonitrile; mobile phase B, distilled water; by a gradient method

A calibration curve was calculated using a solution of nafamostat mesilate, argatroban, or trisodium citrate dissolved in physiological saline. Each measurement was made at N = 3, and the average of the values obtained was used as the anti-coagulant concentration of the extract liquid. The amount of the anti-coagulant per g of dry mass of the hollow fiber membrane was calculated using the hollow fiber membranes 1 to 3, the hollow fiber membranes 13 to 17, and the hollow fiber membranes 27 to 28 in accordance with the following Formula (2), and using the hollow fiber membranes 4 to 11 and the hollow fiber membranes 18 to 26 in accordance with the following Formula (3).

Amount of anti-coagulant per g of dry mass of hollow fiber membrane (μmol/g) = (heparin concentration (IU/mL) of extract liquid × amount (mL) of extract liquid × 8 (μg/IU) / molecular weight (g/mol)) of heparin / mass (g) of hollow fiber membrane     Formula (2)

Amount of anti-coagulant per g of dry mass of hollow fiber membrane (μmol/g) = (anti-coagulant concentration (μmol/mL) of extract liquid × amount (mL) of extract liquid / mass (g) of hollow fiber membrane)     Formula (3)

In this regard, a value of 8 μg/IU was employed as the mass of heparin sodium per unit, and a value of 12,000 g/mol was employed as the molecular weight of heparin was. In addition, a value rounded to two decimal places was used as the amount of the anti-coagulant per g of dry mass of the hollow fiber membrane.

<Measurement of ratio of adsorption of IL-8>

Into a polypropylene-made container, 38 hollow fiber membranes, 4 cm each, were placed. To this container, a fetal bovine serum (hereinafter referred to as "FBS") prepared so as to have an IL-8 concentration at 2000 pg/mL was added to make up 88 mL with respect to a 1 cm$^3$ hollow fiber membrane. The materials were mixed by inversion in an incubator at 37°C for 1 hour. Then, the IL-8 concentration of the FBS was measured using Quantikine ELISA Human IL-8/CXCL8 (Catalog No. D8000C, R&D Systems, Inc.). From the IL-8 concentrations before the mixing by inversion and after the mixing by inversion, the ratio of adsorption of IL-8 was calculated in accordance with the following Formula (4).

Ratio of adsorption of IL-8 (%) = {IL-8 concentration (pg/mL) before mixing by inversion - IL-8 concentration (pg/mL) after mixing by inversion} / IL-8 concentration (pg/mL) before mixing by inversion ×100     Formula (4)

Here, a value rounded off to whole numbers was used as the ratio of adsorption of IL-8.

<Measurement of ratio of removal of activated leukocyte under first conditions>

A healthy human volunteer blood supplemented with LPS to a level of 70 EU/mL was shaken at 65 rpm at 37°C for 30 minutes to attain activation. The blood activated was made to pass at a flow rate of 0.63 mL/minute through the hollow fiber membrane module, and blood samples were collected at the inlet and outlet of the hollow fiber membrane module. The sample collected at the outlet of the hollow fiber membrane module was a sample collected after the blood was made to pass through the module for 6.5 minutes, taking the point of time when blood flowed into the hollow fiber membrane module as 0 minute. The sample collected was measured using an automated multichannel hematology analyzer, and the ratio of removal of activated leukocytes through the hollow fiber membrane under the first conditions was determined using the following Formula (5).

Ratio (%) of removal of activated leukocyte under first conditions = (activated leukocyte concentration of blood at outlet of hollow fiber membrane module (cells/μL)) / (activated leukocyte concentration of blood at inlet of hollow fiber membrane module (cells/μL))     Formula (5)

Here, a value rounded off to whole numbers was used as the ratio of removal of activated leukocyte.

<Measurement of water permeability under first conditions>

The insides of the hollow fiber membranes of the hollow fiber membrane module were filled with water. A hydraulic pressure of 100 mmHg was applied to the insides of the hollow fiber membranes for 15 minutes. The amount of filtration of water that flowed out of the hollow fiber membranes per unit time was measured. The water permeability under the first conditions was calculated in accordance with the following Formula (6).

Water permeability (L/h/m$^2$/mmHg) under first conditions = amount of filtration (L) / time (h) / inner surface areas (m$^2$) of hollow fiber membranes / hydraulic pressure (mmHg)　　　Formula (6)

<Measurement of water permeability after passage of fresh bovine blood and calculation of ratio of maintenance of water permeability under first condition>

Using the hollow fiber membrane module used in "Measurement of water permeability under first conditions" above, a blood passage test was performed using fresh bovine blood (hematocrit, 30%; the total protein concentration, 6.0 g/dL). A circuit was connected to each of the inlet and outlet of the hollow fiber membrane module, and fresh bovine blood kept at 37°C in a water bath was made to pass through at 100 mL/minute for 2 hours. After the passage was stopped, physiological saline was made to pass at 100 mL/minute for 5 minutes through the inside of the hollow fiber membrane module to wash the inside. Subsequently, the water permeability after the passage of the fresh bovine blood was measured by the same method as in "Measurement of water permeability under first conditions" above. The ratio of maintenance of water permeability under the first conditions was calculated in accordance with the following Formula (7).

Ratio (%) of maintenance of water permeability under first conditions = water permeability (L/h/m$^2$/mmHg) after passage of fresh bovine blood / water permeability (L/h/m$^2$/mmHg) under first conditions $\times$ 100　　　Formula (7)

Here, a value rounded off to whole numbers was used as the value of the ratio of maintenance of water permeability under the first conditions.

[Example 1]

"sepXiris" (registered trademark: manufactured by Baxter Healthcare Corporation), which is a hollow fiber membrane composed of an acrylonitrile/sodium methallylsulfonate copolymer, and having polyethyleneimine in the surface thereof, was unraveled using a pipe cutter. Then, 124 hollow fiber membranes taken out were bundled, and packed into a housing having a diameter of approximately 5 mm and a length of 10 cm. Both ends were fixed to the housing with the epoxy-resin chemical-reaction adhesive "QUICK MENDER" (registered trademark: manufactured by Konishi Co., Ltd.). After the adhesive was hardened, the end faces were cut open, whereby a hollow fiber membrane module was produced. Then, through the inside of the hollow fiber membranes of the module, an aqueous 50 mass% DMSO solution of 30°C was made to pass at a flow rate of 0.6 mL/minute for 1.5 hours, 50 mass% DMSO physiological saline having heparin sodium (manufactured by AY Pharmaceuticals Co., Ltd.) dissolved at a concentration of 0.1 mg/mL was made to pass at a flow rate of 0.6 mL/minute for 30 minutes, physiological saline having heparin sodium dissolved at a concentration of 0.1 mg/mL was made to pass at a flow rate of 0.6 mL/minute for 30 minutes, and physiological saline was made to pass at a flow rate of 0.6 mL/minute for 5 minutes, in this order, to afford a hollow fiber membrane module 1 and a hollow fiber membrane 1.

[Example 2]

A hollow fiber membrane module 2 and a hollow fiber membrane 2 were obtained by performing the same operation as in Example 1 except that the heparin sodium concentration of each of the 50 mass% DMSO physiological saline and the physiological saline each having heparin dissolved therein was changed to 0.5 mg/mL.

[Example 3]

A hollow fiber membrane module 3 and a hollow fiber membrane 3 were obtained by performing the same operation as in Example 1 except that the heparin sodium concentration of each of the 50 mass% DMSO physiological saline and the physiological saline each having heparin dissolved therein was changed to 0.04 mg/mL.

[Example 4]

A hollow fiber membrane module 4 and a hollow fiber membrane 4 were obtained by performing the same operation as in Example 1 except that 50 mass% DMSO physiological saline and physiological saline each having, in place of heparin sodium, nafamostat mesilate (manufactured by Nichi-Tko Pharmaceutical Co., Ltd.) dissolved at a concentration of 10 mg/mL were used.

[Example 5]

A hollow fiber membrane module 5 and a hollow fiber membrane 5 were obtained by performing the same operation as in Example 4 except that the nafamostat mesilate concentration of each of the 50 mass% DMSO physiological saline and the physiological saline each having nafamostat mesilate dissolved therein was changed to 50 mg/mL.

[Example 6]

A hollow fiber membrane module 6 and a hollow fiber membrane 6 were obtained by performing the same operation as in Example 4 except that the nafamostat mesilate concentration of each of the 50 mass% DMSO physiological saline and the physiological saline each having nafamostat mesilate dissolved therein was changed to 5 mg/mL.

[Example 7]

A hollow fiber membrane module 7 and a hollow fiber membrane 7 were obtained by performing the same operation as in Example 1 except that 50 mass% DMSO physiological saline and physiological saline each having, in place of heparin sodium, argatroban (manufactured by Nichi-Iko Pharmaceutical Co., Ltd.) dissolved at a concentration of 50 mg/mL were used.

[Example 8]

A hollow fiber membrane module 8 and a hollow fiber membrane 8 were obtained by performing the same operation as in Example 7 except that the argatroban concentration of each of the 50 mass% DMSO physiological saline and the physiological saline each having argatroban dissolved therein was changed to 100 mg/mL.

[Example 9]

A hollow fiber membrane module 9 and a hollow fiber membrane 9 were obtained by performing the same operation as in Example 1 except that 50 mass% DMSO physiological saline and physiological saline each having, in place of heparin sodium, trisodium citrate (manufactured by Fujifilm Wako Pure Chemical Corporation) dissolved at a concentration of 0.04 mg/mL were used.

[Example 10]

A hollow fiber membrane module 10 and a hollow fiber membrane 10 were obtained by performing the same operation as in Example 9 except that the trisodium citrate concentration of each of the 50 mass% DMSO physiological saline and the physiological saline each having trisodium citrate dissolved therein was changed to 0.1 mg/mL.

[Example 11]

A hollow fiber membrane module 11 and a hollow fiber membrane 11 were obtained by performing the same operation as in Example 9 except that the citric acid concentration of each of the 50 mass% DMSO physiological saline and the physiological saline each having citric acid dissolved therein was changed to 0.01 mg/mL.

[Comparative Example 1]

A hollow fiber membrane module 12 and a hollow fiber membrane 12 were obtained by performing the same operation as in Example 1 except that none of the aqueous 50 mass% DMSO solution, the 50 mass% DMSO physiological saline having heparin sodium dissolved at a concentration of 0.1 mg/mL, the physiological saline having heparin sodium dissolved at a concentration of 0.1 mg/mL, and the physiological saline was made to pass through the hollow fiber membrane module.

[Comparative Example 2]

A hollow fiber membrane module 13 and a hollow fiber membrane 13 were obtained by performing the same operation as in Example 3 except that none of the aqueous 50 mass% DMSO solution and the 50 mass% DMSO physiological saline having heparin sodium dissolved at a concentration of 0.1 mg/mL was made to pass.

[Comparative Example 3]

A hollow fiber membrane module 14 and a hollow fiber membrane 14 were obtained by performing the same operation as in Comparative Example 2 except that the heparin sodium concentration of the physiological saline was changed to 1.0 mg/mL.

Using the resulting hollow fiber membrane modules 1 to 14 and hollow fiber membranes 1 to 14, measurements were made of the arithmetic-average roughness (Ra), the amount of the anti-coagulant, the ratio of adsorption of IL-8, the ratio of removal of activated leukocytes under the first conditions, the water permeability under the first conditions, the water permeability after passage of fresh bovine blood, and the ratio of maintenance of water permeability under the first conditions. The measurement results are shown in Table 1.

[Table 1]

| Examples | Arithmetic-average roughness (Ra) [μm] | Anti-coagulant | Amount of anti-coagulant [μmol/g] | Ratio of adsorption of IL-8 [%] | Ratio of removal of activated leukocytes under first conditions [%] | Water permeability under first conditions [L/h/m$^2$/mmHg] | Water permeability after passage of fresh bovine blood [L/h/m$^3$mmHg] | Ratio of maintenance of water permeability under first conditions [%] |
|---|---|---|---|---|---|---|---|---|
| Example 1 | 0.55 | heparin | 0.30 | 80 | 40 | 1.3 | 1.1 | 89 |
| Example 2 | 0.51 | | 0.60 | 65 | 38 | 1.4 | 1.3 | 95 |
| Example 3 | 0.54 | | 0.20 | 79 | 41 | 1.4 | 1.0 | 71 |
| Example 4 | 0.55 | nafamostat mesilate | 2.00 | 75 | 45 | 1.3 | 0.9 | 70 |
| Example 5 | 0.54 | | 6.80 | 71 | 46 | 1.2 | 0.9 | 78 |
| Example 6 | 0.51 | | 1.37 | 78 | 40 | 1.3 | 0.8 | 60 |
| Example 7 | 0.57 | argatroban | 11.00 | 70 | 43 | 1.4 | 1.1 | 76 |
| Example 8 | 0.49 | | 40.00 | 62 | 39 | 1.2 | 1.0 | 83 |
| Example 9 | 0.53 | citric acid | 0.17 | 77 | 42 | 1.3 | 0.9 | 70 |
| Example 10 | 0.49 | | 0.40 | 75 | 38 | 1.3 | 1.3 | 100 |
| Example 11 | 0.54 | | 0.07 | 75 | 39 | 1.3 | 0.6 | 45 |
| Comparative Example 1 | 0.50 | none | 0.00 | 78 | 40 | 1.3 | 0.2 | 15 |
| Comparative Example 2 | 0.03 | heparin | 0.09 | 85 | 3 | 1.5 | 0.7 | 45 |
| Comparative Example 3 | 0.02 | | 0.16 | 80 | 10 | 1.4 | 0.6 | 43 |

<Measurement of ratio of removal of activated leukocyte under second conditions>

The same operation as for the measurement of the ratio of removal of activated leukocytes under the first conditions was performed except that the blood passage time was changed to 3 minutes, taking the point of time when blood flowed into the hollow fiber membrane module as 0 minute. The ratio of removal of activated leukocytes under the second conditions was calculated in accordance with the following Formula (8).

Ratio (%) of removal of activated leukocyte under second conditions = (activated leukocyte concentration (cells/μL) of blood at outlet of hollow fiber membrane module) / (activated leukocyte concentration (cells/μL) of blood at inlet of hollow fiber membrane module)          Formula (8)

<Measurement of water permeability under second conditions>

The same operation as for the measurement of the water permeability under the first conditions was performed except that the filtration time was changed to 6 minutes, and that the hydraulic pressure was changed to 120 mmHg. The water permeability under the second conditions was calculated in accordance with the following Formula (9).

Water permeability (L/h/m$^2$/mmHg) under second conditions = amount of filtration (L) / time (h) / inner surface area (m$^2$) of hollow fiber membrane / hydraulic pressure (mmHg)          Formula (9)

<Measurement of water permeability and calculation of ratio of maintenance of water permeability under second condition, after measurement of ratio of removal of activated leukocytes>

Blood was made to pass through the hollow fiber membrane module used in "Measurement of ratio of removal of activated leukocyte under second conditions" above. The module was washed with physiological saline at 0.63 mL/minute for 10 minutes. Then a solution of 2.5% glutaraldehyde/2% paraformaldehyde in PBS was packed in the hollow fiber membrane module, and left to stand overnight. Subsequently, the water permeability after the measurement of the ratio of removal of activated leukocytes was measured by the same method as in "Measurement of water permeability under second conditions" above. The ratio of maintenance of water permeability under the second conditions was calculated in accordance with the following Formula (10).

Ratio (%) of maintenance of water permeability under second conditions = water permeability (L/h/m$^2$/mmHg) after measurement of ratio of removal of activated leukocyte / water permeability (L/h/m$^2$/mmHg) under second conditions $\times$ 100          Formula (10)

Here, a value rounded off to whole numbers was used as the value of the ratio of maintenance of water permeability under the second conditions

[Example 12]

A hollow fiber membrane module 15 and a hollow fiber membrane 15 were obtained by performing the same operation as in Example 1 except that the number of the hollow fiber membranes packed in the housing was changed to 40, and that the length was changed to 12 cm.

[Example 13]

A hollow fiber membrane module 16 and a hollow fiber membrane 16 were obtained by performing the same operation as in Example 2 except that the number of the hollow fiber membranes packed in the housing was changed to 40, and that the length was changed to 12 cm.

[Example 14]

A hollow fiber membrane module 17 and a hollow fiber membrane 17 were obtained by performing the same operation as in Example 3 except that the number of the hollow fiber membranes packed in the housing was changed to 40, and that the length was changed to 12 cm.

[Example 15]

A hollow fiber membrane module 18 and a hollow fiber membrane 18 were obtained by performing the same operation as in Example 4 except that the number of the hollow fiber membranes packed in the housing was changed to 40, and that the length was changed to 12 cm.

[Example 16]

A hollow fiber membrane module 19 and a hollow fiber membrane 19 were obtained by performing the same operation as in Example 5 except that the number of the hollow fiber membranes packed in the housing was changed to 40, and that the length was changed to 12 cm.

[Example 17]

A hollow fiber membrane module 20 and a hollow fiber membrane 20 were obtained by performing the same operation as in Example 6 except that the number of the hollow fiber membranes packed in the housing was changed to 40, and that the length was changed to 12 cm.

[Example 18]

A hollow fiber membrane module 21 and a hollow fiber membrane 21 were obtained by performing the same operation as in Example 7 except that the number of the hollow fiber membranes packed in the housing was changed to 40, and that the length was changed to 12 cm.

[Example 19]

A hollow fiber membrane module 22 and a hollow fiber membrane 22 were obtained by performing the same operation as in Example 8 except that the number of the hollow fiber membranes packed in the housing was changed to 40, and that the length was changed to 12 cm.

[Example 20]

A hollow fiber membrane module 23 and a hollow fiber membrane 23 were obtained by performing the same operation as in Example 9 except that the number of the hollow fiber membranes packed in the housing was changed to 40, and that the length was changed to 12 cm.

[Example 21]

A hollow fiber membrane module 24 and a hollow fiber membrane 24 were obtained by performing the same operation as in Example 10 except that the number of the hollow fiber membranes packed in the housing was changed to 40, and that the length was changed to 12 cm.

[Example 22]

A hollow fiber membrane module 25 and a hollow fiber membrane 25 were obtained by performing the same operation as in Example 11 except that the number of the hollow fiber membranes packed in the housing was changed to 40, and that the length was changed to 12 cm.

[Comparative Example 4]

A hollow fiber membrane module 26 and a hollow fiber membrane 26 were obtained by performing the same operation as in Comparative Example 1 except that the number of the hollow fiber membranes packed in the housing was changed to 40, and that the length was changed to 12 cm.

[Comparative Example 5]

A hollow fiber membrane module 27 and a hollow fiber membrane 27 were obtained by performing the same operation as in Comparative Example 2 except that the number of the hollow fiber membranes packed in the housing was changed to 40, and that the length was changed to 12 cm.

[Comparative Example 6]

A hollow fiber membrane module 28 and a hollow fiber membrane 28 were obtained by performing the same operation as in Comparative Example 3 except that the number of the hollow fiber membranes packed in the housing was changed to 40, and that the length was changed to 12 cm.

Using the resulting hollow fiber membrane modules 15 to 28 and hollow fiber membranes 15 to 28, measurements were made of the ratio of removal of activated leukocytes under the second conditions, the water permeability under the second conditions, the water permeability after measurement of the ratio of removal of activated leukocytes, and the ratio of maintenance of water permeability under the second conditions. The measurement results are shown in Table 2.

[Table 2]

| Examples | Anti-coagulant | Ratio of removal of activated leukocytes under second conditions [%] | Water permeability under second conditions [L/h/m²/mmHg] | Water permeability after measurement of ratio of removal of activated leukocytes [L/h/m²/mmHg] | Ratio of maintenance of water permeability under second conditions [%] |
|---|---|---|---|---|---|
| Example 12 | heparin | 3.2 | 0.029 | 0.025 | 86 |
| Example 13 | | 2.6 | 0.029 | 0.027 | 93 |
| Example 14 | | 5.9 | 0.029 | 0.025 | 86 |
| Example 15 | nafamostat mesilate | 4.1 | 0.030 | 0.024 | 80 |
| Example 16 | | 5.2 | 0.028 | 0.023 | 82 |
| Example 17 | | 3.8 | 0.029 | 0.024 | 83 |
| Example 18 | argatroban | 4.5 | 0.030 | 0.026 | 87 |
| Example 19 | | 3.7 | 0.028 | 0.022 | 79 |
| Example 20 | citric acid | 4.4 | 0.029 | 0.023 | 79 |
| Example 21 | | 3.6 | 0.030 | 0.029 | 97 |
| Example 22 | | 3.7 | 0.029 | 0.025 | 86 |
| Comparative Example 4 | none | 2.5 | 0.027 | 0.013 | 48 |
| Comparative Example 5 | heparin | 0.2 | 0.029 | 0.015 | 52 |
| Comparative Example 6 | | 0.7 | 0.028 | 0.013 | 46 |

Explanation of Symbols

100    Curved outline
101    Average line

**Claims**

1. A hollow fiber membrane comprising an anti-coagulant, and having a surface that comes into contact with a liquid to be treated, wherein said surface has an arithmetic-average roughness (Ra) of 0.10 to 0.80 μm in the radial direction.

2. The hollow fiber membrane according to claim 1, having an amount of said anti-coagulant per g of dry mass of 0.07 to 50.00 μmol.

3. The hollow fiber membrane according to claim 1, having an amount of said anti-coagulant per g of dry mass of 0.27 to 30.00 $\mu$mol.

4. The hollow fiber membrane according to claim 1 or 2, wherein said surface that comes in contact with said liquid to be treated comprises an amino group present therein.

5. The hollow fiber membrane according to claim 1 or 2, wherein the material of said hollow fiber membrane is a copolymer of: a hydrophobic monomer selected from the group consisting of acrylonitrile, methyl methacrylate, and styrene; and a hydrophilic monomer selected from the group consisting of methallylsulfonic acid, styrenesulfonic acids and salts thereof.

6. The hollow fiber membrane according to claim 1 or 2, for use in hemocatharsis.

7. The hollow fiber membrane according to claim 1 or 2, for use in the adsorptive removal of activated leukocytes and inflammatory cytokines.

8. A hollow fiber membrane module comprising said hollow fiber membrane according to claim 1 or 2.

9. The hollow fiber membrane module according to claim 8, for use in hemocatharsis.

Fig.1

## INTERNATIONAL SEARCH REPORT

| International application No. |
| --- |
| **PCT/JP2024/006168** |

### A. CLASSIFICATION OF SUBJECT MATTER

*A61M 1/18*(2006.01)i; *A61M 1/36*(2006.01)i; *B01D 63/02*(2006.01)i; *B01D 67/00*(2006.01)i; *B01D 69/02*(2006.01)i; *B01D 69/08*(2006.01)i; *B01D 71/80*(2006.01)i

FI: A61M1/18 500; A61M1/36 165; A61M1/36 153; B01D63/02; B01D69/02; B01D69/08; B01D67/00; B01D71/80

According to International Patent Classification (IPC) or to both national classification and IPC

### B. FIELDS SEARCHED

Minimum documentation searched (classification system followed by classification symbols)

A61M1/18; A61M1/36; B01D63/02; B01D67/00; B01D69/02; B01D69/08; B01D71/80

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| Y | WO 2019/049961 A1 (TORAY INDUSTRIES, INC.) 14 March 2019 (2019-03-14) paragraphs [0001], [0013]-[0129] | 1-9 |
| Y | WO 2022/220292 A1 (TORAY INDUSTRIES, INC.) 20 October 2022 (2022-10-20) paragraphs [0015]-[0126] | 1-9 |

☐ Further documents are listed in the continuation of Box C.   ☑ See patent family annex.

| | | |
| --- | --- | --- |
| * | Special categories of cited documents: | |
| "A" | document defining the general state of the art which is not considered to be of particular relevance | |
| "D" | document cited by the applicant in the international application | |
| "E" | earlier application or patent but published on or after the international filing date | |
| "L" | document which may throw doubts on priority claim(s) or which is cited to establish the publication date of another citation or other special reason (as specified) | |
| "O" | document referring to an oral disclosure, use, exhibition or other means | |
| "P" | document published prior to the international filing date but later than the priority date claimed | |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
| --- | --- |
| **19 March 2024** | **02 April 2024** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

## INTERNATIONAL SEARCH REPORT
### Information on patent family members

International application No.

**PCT/JP2024/006168**

| Patent document cited in search report | | | Publication date (day/month/year) | Patent family member(s) | | | Publication date (day/month/year) |
|---|---|---|---|---|---|---|---|
| WO | 2019/049961 | A1 | 14 March 2019 | US | 2020/0246776 | A1 | |
| | | | | paragraphs [0001], [0035]-[0154] | | | |
| | | | | EP | 3679966 | A1 | |
| | | | | CN | 111065425 | A | |
| | | | | KR | 10-2020-0051586 | A | |
| | | | | CA | 3073500 | A1 | |
| WO | 2022/220292 | A1 | 20 October 2022 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021066152 A
- JP 2020516424 A
- JP 2022125040 A